# DEMANDE DE BREVET EUROPEEN

(11) **EP 4 850 636 A1**
(43) Date de publication de la demande: **09.12.2026**
(21) Numéro de dépôt: 26181126.9
(22) Date de dépôt: 26.05.2026
(51) Int. Cl.: A61F 2/38, A61F 2/30

(54) **ENSEMBLE D'IMPLANTS TIBIAUX ET/OU FEMORAUX DE PROTHESE DE GENOU**

(30) Priorité: 03.06.2025 FR 2505964
(71) Demandeur: XNOV Medical Technology SA, 2900 Porrentruy (CH)
(72) Inventeur: AUCOUTURIER, Thierry, 68026 Colmar (FR); BIEGUN, Jean-François, 2926 Boncourt (CH); BIEGUN, Frédérique, 2926 Boncourt (CH); DANGIN, Antoine, 42170 Saint-Juste-Saint-Robert (FR); DURAND, Jean-Christophe, 69116 Limonest (FR); LARDANCHER, Jean-François, 80680 Sains-en-Amienois (FR); LOEHLE, Pascal, 2904 Bressaucourt (CH); MULLIEZ, Arnaud, 59000 Lille (FR); SIGWALT, Loïc, 38500 Voiron (FR); WAJSFISZ, Anthony, 75009 Paris (FR)
(74) Mandataire: Eidelsberg, Olivier Nathan

(57) **Abrégé**

Gamme (T1, T2, T3) d'embases tibiales, comportant au moins deux embases (2), notamment métalliques, pour un composant tibial d'une prothèse de genou, chacune des embases étant destinée à être fixée seule, sans les autres, au tibia, au niveau d'une coupe osseuse du tibia, les embases étant de dimensions différentes les unes des autres et l'embase fixée choisie étant celle dont la dimension s'adapte le mieux à celle de la coupe osseuse du tibia, les embases ayant chacune des moyens de fixation (3) respectifs destinés à la fixation de l'embase respective au tibia au niveau de la coupe osseuse du tibia, chaque embase ayant une surface supérieure dans laquelle est formée une cavité de réception (4) destinée à recevoir un insert tibial, les cavités de réception des embases ayant des formes et dimensions identiques, de sorte à pouvoir recevoir en leur sein, notamment à complémentarité de forme, un même insert tibial.

## Description

La présente invention se rapporte à une gamme d'au moins deux composants tibiaux pour une prothèse de genou et/ou une gamme d'au moins deux composant fémoraux pour une prothèse de genou, ainsi qu'à un kit pour former une prothèse de genou comportant au moins une de ces deux gammes et à un procédé pour former une prothèse de genou avant sa pose.

Un composant tibial dans une prothèse de genou se compose généralement d'une embase tibiale, classiquement en métal, fixée sur le plateau tibial de l'os et d'un insert en un matériau moins dur, notamment en polyéthylène (PE) ou analogue, reposant sur l'embase et agissant comme un coussinet lisse entre l'embase tibiale et un ou des condyles d'un composant fémoral de la prothèse. Ensemble, l'embase et l'insert permettent une articulation fluide du genou en remplaçant les surfaces articulaires endommagées.

Un composant fémoral se compose en général d'une part d'une partie supérieure en forme de coin destiné à porter contre le fémur, des moyens de fixation, notamment des plots d'ancrage, faisant saillie de la partie supérieure en forme de coin pour une fixation ou ancrage au fémur, et d'autre part, du côté opposé, d'un ou deux condyles de forme incurvée destiné(s) à venir en contact avec une face supérieure de l'insert.

Les composants tibiaux traditionnels nécessitent souvent des embases et des inserts de tailles et de formes spécifiques adaptés aux différents genoux. Cette spécificité nécessite des références multiples pour chaque configuration, augmentant ainsi les coûts de production, de stockage, et la complexité de l'utilisation en salle d'opération. Les chirurgiens peuvent rencontrer des difficultés de choix de taille en peropératoire, ce qui peut rallonger la durée de l'intervention et augmenter le risque d'erreurs.

Les composants fémoraux traditionnels nécessitent souvent des coupes osseuses différentes de forme spécifiques adaptés aux différents genoux en fonction de la taille des composants choisie. Cette spécificité augmente la complexité de l'utilisation et rallonge le temps en salle d'opération. Les chirurgiens peuvent rencontrer des difficultés de choix de taille en peropératoire, ce qui peut rallonger la durée de l'intervention et augmenter le risque d'erreurs.

De US2006/0190086A1, on connait également une prothèse de genou comportant un composant tibial constitué d'un ensemble de trois embases tibiales, respectivement médiale, latérale et centrale, fixées ensembles, les unes à côté des autres, à un unique tibia au niveau d'une coupe tibiale de ce dernier, et soit trois inserts de formes différentes étant insérés dans des cavités respectives des trois embases, soit un insert unique comportant trois saillies correspondant aux trois cavités des embases étant inséré dans les trois cavités, le côté opposé ou supérieur de l'insert coopérant avec des condyles d'un composant fémoral de la prothèse.

La présente invention vise à surmonter les inconvénients de l'art antérieur, et notamment à simplifier le choix de la prothèse à poser et à diminuer la durée de l'opération de pose de la prothèse en proposant, suivant un premier aspect, une gamme d'embases tibiales telle que définie à la revendication 1 et/ou une gamme de composants fémoraux telle que définie à la revendication 8.

La présente invention se rapporte également à un ensemble comportant une gamme d'embases tibiales suivant la revendication 1 et un insert en un matériau moins dur, notamment en PE, caractérisé en ce que les cavités de réception des embases ont des formes et dimensions identiques, de sorte à pouvoir recevoir l'insert en leur sein, notamment à complémentarité de forme.

L'insert est ainsi adapté à plusieurs tailles d'embases et les composant fémoraux de la gamme sont tous fixés à un fémur avec la même orientation, quelle que soit la taille du fémur, ce qui réduit le nombre d'éléments à produire et à stocker, ainsi qu'à avoir à disposition en salle d'opération, évitant ainsi l'encombrement pour le chirurgien. De plus, cela réduit les erreurs de tailles et le choix pour le chirurgien avant l'opération. La découpe fémorale est unique pour toutes les tailles et le chirurgien peut sélectionner la taille la plus adaptée après avoir réalisé les opérations de coupe.

De préférence, l'insert est en un matériau déformable élastiquement, notamment en un polymère, notamment en PE ou PE hautement réticulé.

De préférence, l'insert est fixé dans la cavité de l'embase par encliquetage. Par exemple, deux saillies peuvent être formées dans la paroi latérale de la cavité de réception des embases et deux rainures conjuguées sont formées dans l'insert.

L'encliquetage de l'insert permet une mise en place facilitée et sans erreur possible et il n'est pas prévu de languette déformable qui peut se tordre lors de la mise place. L'encliquetage est réalisé par la déformation élastique de l'ensemble de l'insert polymère.

De préférence, les saillies de l'embase sont parallèles et forment un rail qui permet l'insertion toujours dans le bon axe de l'insert. Si l'insert polymère n'est pas bien positionnée, l'encliquetage n'est pas possible et l'insert polymère n'est pas endommagée.

De préférence, l'insert peut être inséré dans la cavité de réception d'une embase pour jambe droite ou gauche. L'embase métallique peut être asymétrique pour garantir une meilleure couverture osseuse possible. Le même insert est utilisable avec l'embase pour jambe gauche ou droite.

De préférence, chaque embase a une surface supérieure délimitée par un bord périphérique extérieur respectif, chaque cavité est délimitée par une paroi périphérique ayant un bord périphérique supérieur, les dimensions des bords périphériques extérieurs des embases, notamment leurs extensions périphériques, étant différentes les unes aux autres, tandis que les bords périphériques supérieurs des cavités ont des dimensions identiques les unes aux autres.

La présente invention se rapporte également à un kit pour former une prothèse de genou comportant un ensemble suivant la revendication 2 et un composant fémoral, ainsi qu'à un kit pour former une prothèse de genou comportant un composant tibial et une gamme de composants fémoraux suivant la revendication 8, ainsi qu'à un kit pour former une prothèse de genou comportant un ensemble suivant la revendication 2 et une gamme de composant fémoraux suivant la revendication 8.

La présente invention se rapporte également à un procédé pour former une prothèse de genou en vue de sa pose, comportant les étapes dans laquelle on choisit parmi les embases tibiales d'une gamme d'embases tibiales suivant l'une des revendications 1 à 7 celle dont la dimension s'adapte le mieux ou correspond le plus à celle de la section du tibia destinée à être découpée et/ou on choisit parmi les composants fémoraux de la gamme de composants fémoraux suivant l'une des revendications 8 à 9, celui dont les dimensions des faces supérieures en forme de coin et/ou des faces inférieures formant un ou des condyles s'adapte le mieux ou correspond le plus aux dimensions d'au moins deux coupes osseuses destinées à être réalisées sur le fémur et faisant entre elles ledit angle de coupe.

A titre d'exemple uniquement, on décrit à l'aide des dessins annexés un mode de réalisation préféré de l'invention.
La figure 1 est une vue en perspective d'un ensemble de deux composants tibiaux droit et gauche suivant l'invention ;
La figure 2 est une vue en coupe d'un composant tibial pendant l'encliquetage de l'insert dans son embase, issue d'une gamme d'embase de l'ensemble de la figure 1 ;

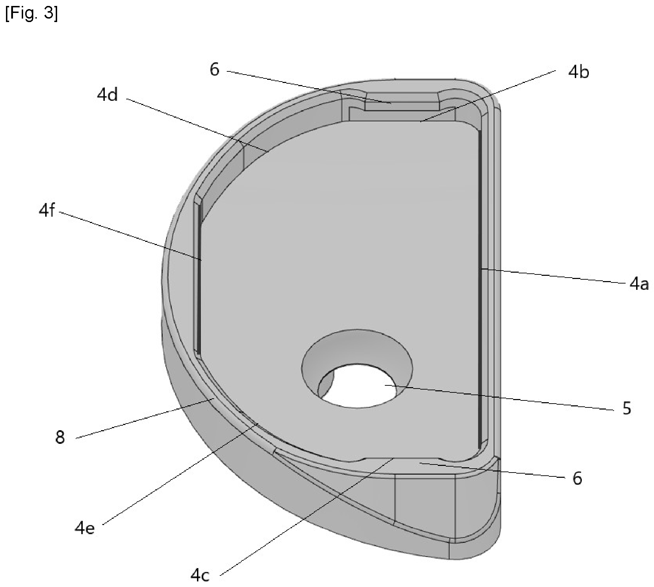

La figure 3 est une vue de dessus d'une embase de la gamme d'embases de l'ensemble de figure 1 ;

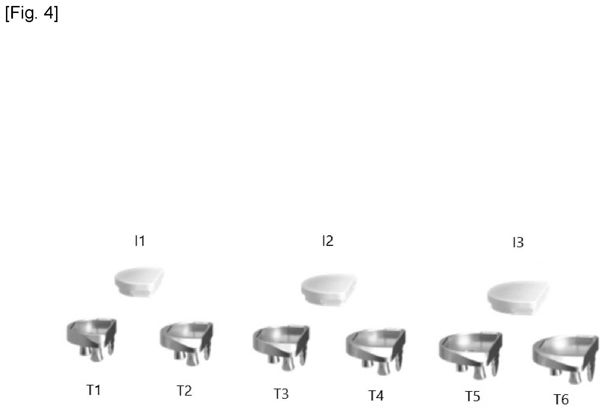

La figure 4 est une vue en perspective d'une gamme de plusieurs ensembles d'implant tibiaux suivant l'invention ; et
La figure 5 est une vue de profil d'un fémur sur lequel ont été représentés fixés les différentes tailles de composants fémoraux d'un kit de composants fémoraux suivant l'invention.

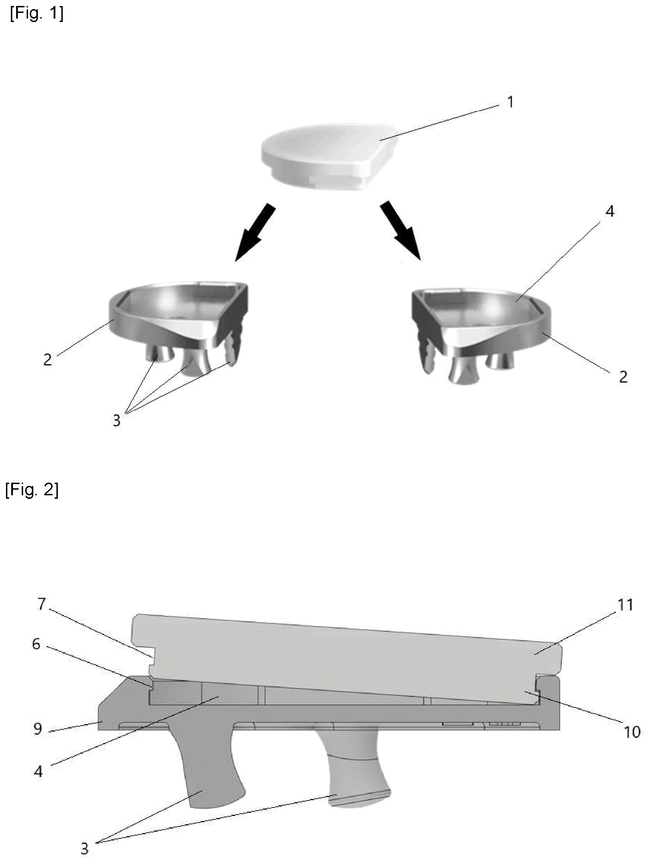

A la figure 1, il est représenté un ensemble comportant un insert 1 en polyéthylène, ou PE, déformable élastiquement et une gamme de deux embases 2 métalliques pour jambes droite et gauche. Chaque embase 2 tibiale de la gamme présente une forme et des dimensions déterminées pour s'adapter parfaitement à un tibia correspondant. Chaque embase 2, en coupe longitudinale, a une forme asymétrique ayant un tronçon rectiligne et un tronçon en arc de cercle, de sorte qu'elle peut être fixée à un tibia gauche ou droit. Une gamme comporte deux embases 2 de même forme mais de dimensions différentes.

Chaque embase 2 comporte une surface inférieure de laquelle font saillie trois éperons 3 latéraux et une surface supérieure dans laquelle est creusée une cavité 4 conformée pour recevoir l'insert 1 et orientée de sorte que ledit insert 1 soit toujours à l'aplomb de la trajectoire de contact fémoro-tibial. La fixation de l'embase 2 au tibia correspondant s'effectue au moyen d'une vis qui traverse un trou 5 traversant formé dans le fond de la cavité 4 de l'embase 2, tandis que les éperons 3 latéraux assurent le blocage en rotation de ladite embase 2.

La paroi latérale périphérique de la cavité 4 comporte, en coupe longitudinale, un tronçon 4a principal rectiligne, des extrémités duquel s'étendent perpendiculairement, deux tronçons 4b,4c rectilignes parallèles entre eux chacun suivi d'un tronçon 4d,4e en arc de cercle respectif, les tronçons 4d,4e en arc de cercle étant reliés par un deuxième tronçon 4f rectiligne parallèle au tronçon 4a principal. Au niveau de chacun des deux tronçons 4b,4c rectilignes, la paroi latérale périphérique de la cavité 4 comporte deux projections 6 faisant saillie du bord 8 supérieur de la paroi 6 latérale vers l'intérieur de la cavité.

La surface inférieure de l'embase comporte un bord 9 périphérique extérieur.

L'insert 1 comporte une partie 10 inférieure de même conformation, en coupe longitudinale, que celle de la cavité 4, et ayant deux rainures 7 conjuguées aux saillies 6 de la cavité 4, et une partie 11 supérieure de même forme que la partie 10 inférieure mais de dimensions supérieures, qui une fois l'insert 1 dans la cavité 4, dépasse de celle-ci. L'insert 1 étant déformable élastiquement, **il** se plie pour que les saillies 6 de la cavité pénètrent dans les rainures 7 de l'insert 1. Les tronçons rectilignes 4a principal et 4f parallèles assurent un bon alignement de l'insert 1 dans la cavité 4. Ainsi, le même insert 1 peut être inséré dans les deux tailles d'embases 2 de la gamme.

Les différentes dimensions d'embases 2 d'une gamme sont T1 et T2.

Les extensions en longueur des deux bords 9 périphériques extérieurs des deux embases de la gamme sont différentes l'une de l'autre, celle de l'embase de dimension T1 étant plus petite que celle de l'embase de dimension T2.

De même, les surfaces totales des deux surfaces inférieures des deux embases de la gamme sont différentes l'une de l'autre, celle de l'embase de dimension T1 étant plus petite que celle de l'embase de dimension T2.

La dimension de l'insert 1, notamment son extension en longueur dans la direction AP (antéro-postérieur), est égale à I1 pour l'ensemble d'embase T1, T2.

Les embases d'une autre gamme d'embases 2 sont de dimensions T3 et T4, la dimension de l'insert 1, notamment son extension en longueur dans la direction AP (antéro-postérieur), étant égale à I2 pour l'ensemble d'embases T3, T4.

Les embases d'une autre gamme d'embases 2 sont de dimensions T5 et T6, la dimension de l'insert 1, notamment son extension en longueur dans la direction AP (antéro-postérieur), étant égale à I3 pour l'ensemble d'embases T5, T6.

En particulier, les dimensions T1 à T6 vont en augmentant, en particulier les extensions en longueur de leur bord périphérique extérieur, la surface totale de leur surface extérieure et leur extension (I1 à I3) en longueur dans la direction AP.

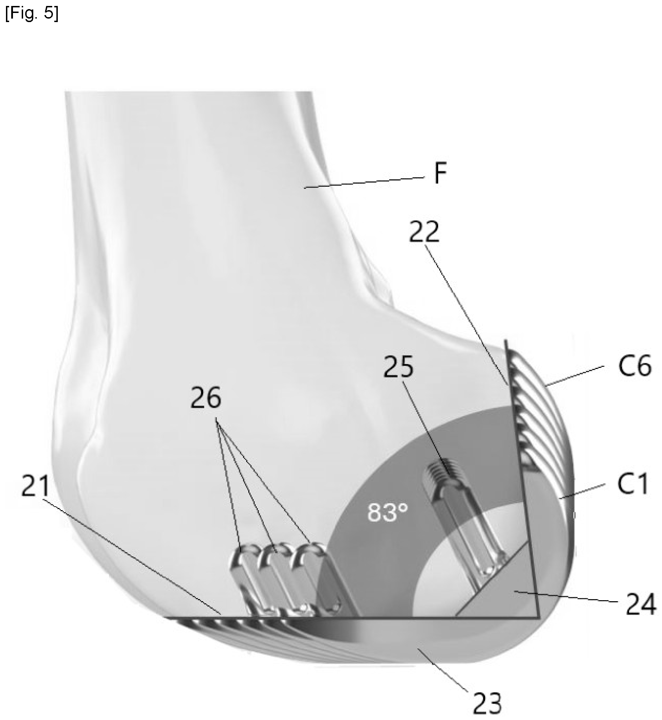

A la figure 5, il est représenté un fémur F sur lequel a été réalisé, par découpe, par exemple à l'aide d'un guide de coupe et d'une lame, comme il est bien connu dans le domaine, deux surfaces de coupe respectivement inférieure et postérieure qui font entre elles un angle de 83°.

En outre, il est représenté six composants fémoraux C1 à C6, seuls C1 et C6 étant indiqués à la figure, le composant C6 étant le composant de la plus grande taille et C1 celui de la plus petite taille.

Chaque composant fémoral C1 à C6 comporte, du côté du fémur ou côté supérieur, une partie supérieure en forme de coin formée de deux surfaces 21, 22 inférieure et postérieure destinées à porter contre les deux surfaces de coupe formées sur le fémur.

Les deux surfaces 21 et 22 font entre elles un angle dit de coupe, notamment de 83°. Les deux surfaces 21 et 22 de chaque composant fémoral C1 à C6 font toutes entre elles ce même angle dit de coupe, notamment 83°.

De l'autre côté, opposé au fémur, chaque composant C1 à C6 comporte deux condyles 23 gauche et droit (seul le droit est visible à la figure) séparé par une région 24 inter-condyle.

Un plot 25 d'ancrage central fait saillie de la région inter-condyle pour s'ancrer dans le fémur. D'autre part, chaque composant C1 à C6 comporte deux plots 26 d'ancrage auxiliaire gauche et droit faisant saillie de la surface 21 inférieure de leur partie supérieure en forme de coin respective, également pour s'ancrer dans le fémur et assurer ainsi avec le plot central la fixation du composant fémoral au fémur F.

Les extensions des surfaces 21 inférieures augmentent du composant fémoral C1 de la plus petite taille au composant fémoral C6 de la plus grande taille.

Les extensions des surfaces 22 postérieures augmentent du composant fémoral C1 de la plus petite taille au composant fémoral C6 de la plus grande taille.

Les distances des plots 26 auxiliaires au plot 25 central augmentent du composant fémoral C1 de la plus petite taille au composant fémoral C6 de la plus grande taille

## Revendications

1. Gamme (T1, T2, T3) d'embases tibiales, comportant au moins deux embases, notamment métalliques, pour un composant tibial d'une prothèse de genou, chacune des embases étant destinée à être fixée seule, sans les autres, au tibia, au niveau d'une coupe osseuse du tibia, les embases étant de dimensions différentes les unes des autres et l'embase fixée choisie étant celle dont la dimension s'adapte le mieux à celle de la coupe osseuse du tibia, les embases ayant chacune des moyens de fixation respectifs destinés à la fixation de l'embase respective au tibia au niveau de la coupe osseuse du tibia, chaque embase ayant une surface supérieure dans laquelle est formée une cavité de réception destinée à recevoir un insert tibial, **caractérisée en ce que** les cavités de réception des embases ont des formes et dimensions identiques, de sorte à pouvoir recevoir en leur sein, notamment à complémentarité de forme, un même insert tibial.

2. Ensemble pour former un composant tibial pour une prothèse de genou, comportant une gamme (T1, T2, T3) suivant la revendication 1 et un insert (1) en un matériau moins dur, notamment en PE, **caractérisé en ce que** les cavités (4) de réception des embases ont des formes et dimensions identiques, de sorte à pouvoir recevoir en leur sein, notamment à complémentarité de forme, l'insert (1).

3. Ensemble suivant la revendication 2, **caractérisé en ce que** l'insert (1) se fixé dans chaque cavité (4) par encliquetage.

4. Ensemble suivant la revendication 3, **caractérisé en ce que** deux (6) saillies sont formées dans la paroi latérale de la cavité (4) de réception des embases (2) et deux rainures (7) conjuguées sont formées dans l'insert (1).

5. Ensemble suivant la revendication 4, **caractérisé en ce que** les deux saillies (6) sont parallèles l'une par rapport à l'autre.

6. Ensemble suivant l'une des revendications 2 à 5, **caractérisé en ce que** l'insert (1) peut être inséré dans la cavité (4) de réception d'une embase (2) pour jambe droite ou gauche.

7. Ensemble suivant l'une des revendications 2 à 6, **caractérisé en ce que** l'embase (2), notamment métallique, est asymétrique.

8. Gamme de composants fémoraux, comportant au moins deux composants (C1, ... , C6) fémoraux pour une prothèse de genou, chaque composant fémoral ayant une face supérieure respective en forme de coin, destinée à porter contre des surfaces de coupe du fémur, et une face inférieure formant un ou des condyles (23) respectifs de forme incurvée, chaque composant fémoral comportant des moyens de fixation au fémur respectifs, notamment des plots d'ancrage, pour la fixation d'au moins deux parois de la face supérieure en forme de coin, faisant entre elles un angle dit de coupe, à au moins deux surfaces respectives formées par au moins deux coupes osseuses du fémur faisant entre elles le même angle dit de coupe, **caractérisée en ce que** les angles dits de coupe respectifs de chacun des au moins deux composants fémoraux sont égaux, tandis que les dimensions des faces supérieures en forme de coin et/ou des faces inférieures formant un ou des condyles varient d'un composant fémoral à l'autre.

9. Gamme suivant la revendication 8, **caractérisée en ce que** les moyens de fixation au fémur font saillie d'au moins une des au moins deux parois de la face supérieure en forme de coin.

10. Kit pour former une prothèse de genou comportant un ensemble pour composant tibial suivant l'une des revendications 2 à 7 et un composant fémoral.

11. Kit pour former une prothèse de genou comportant un composant tibial et une gamme de composants fémoraux suivant l'une des revendications 8 ou 9.

12. Kit pour former une prothèse de genou comportant un ensemble pour composant tibial suivant l'une des revendications 2 à 7 et une gamme de composants fémoraux suivant l'une des revendications 8 ou 9.

13. Procédé pour former une prothèse de genou en vue de sa pose, comportant les étapes dans laquelle on choisit parmi les embases tibiales d'une gamme d'embases tibiales suivant l'une des revendications 1 à 7 celle dont la dimension s'adapte le mieux ou correspond le plus à celle de la section du tibia destinée à être découpée et/ou on choisit parmi les composants fémoraux de la gamme de composants fémoraux suivant l'une des revendications 8 à 9, celui dont les dimensions des faces supérieures en forme de coin et/ou des faces inférieures formant un ou des condyles s'adapte le mieux ou correspond le plus aux dimensions d'au moins deux coupes osseuses destinées à être réalisées sur le fémur et faisant entre elles ledit angle de coupe.
